# EUROPEAN PATENT SPECIFICATION

(11) **EP 3 986 378 B1**
(45) Date of publication and mention of the grant of the patent: **17.12.2025**
(21) Application number: 20733384.0
(22) Date of filing: 11.06.2020
(51) Int. Cl.: A61K 9/06, A61K 38/21, A61K 47/10, A61K 47/36, A61K 9/00

(54) **PHARMACEUTICAL COMPOSITIONS IN THE FORM OF GEL CONTAINING XYLOGLUCAN AND ALCOHOLS FOR THE CONTROLLED RELEASE OF ACTIVE INGREDIENTS**
PHARMAZEUTISCHE ZUSAMMENSETZUNGEN IN FORM VON GEL MIT XYLOGLUCAN UND ALKOHOLEN ZUR KONTROLLIERTEN FREISETZUNG VON WIRKSTOFFEN
COMPOSITIONS PHARMACEUTIQUES SOUS FORME DE GEL CONTENANT DU XYLOGLUCANE ET DES ALCOOLS POUR LA LIBÉRATION CONTRÔLÉE D'INGRÉDIENTS ACTIFS

(30) Priority: 21.06.2019 IT 201900009273
(43) Date of publication of application: 27.04.2022
(73) Proprietor: Alfasigma S.p.A., 40133 Bologna (IT)
(72) Inventor: VISCOMI, Giuseppe Claudio, 40133 Bologna (IT); MAFFEI, Paola, 40133 Bologna (IT); BRUNO, Cristiana, 40133 Bologna (IT); FEDERICI, Mascia, 40133 Bologna (IT); DISPENZA, Clelia, 90128 Palermo (IT); SABATINO, Maria Antonietta, 90128 Palermo (IT)
(86) International application number: PCT/EP2020/066181
(87) International publication number: WO 2020/254179

(56) References cited:
- S. TODARO ET AL: "Radiation Engineering of Xyloglucan Hydrogels", CHEMICAL ENGINEERING TRANSACTIONS, vol. 49, 2016, pages 289 - 294, XP055676514, DOI: 10.3303/CET1649049
- "I materiali biocompatibili per la medicina / Biomaterials for Medicine", 2014, UNIVERSITAS STUDIORUM, article S. PASTA ET AL: "Polysaccharide-based hydrogels for myocardium remodeling", pages: 369 - 370, XP055676486
- K SARADA ET AL: "In-Situ Gelling System: A Review", November 2014 (2014-11-01), pages 76 - 90, XP055676489, Retrieved from the Internet <URL:http://impactfactor.org/PDF/IJCPR/5/IJCPR,Vol5,Issue4,Article2.pdf> [retrieved on 20200313]
- YOSHIAKI YUGUCHI ET AL: "Gelation of xyloglucan in water/alcohol systems", CELLULOSE, vol. 11, no. 2, June 2004 (2004-06-01), Netherlands, pages 203 - 208, XP055645209, ISSN: 0969-0239, DOI: 10.1023/B:CELL.0000025427.60557.40

## Description

### Field of the invention

The present invention relates to gel compositions comprising pharmaceutical active ingredients.

The composition comprises xyloglucan at a concentration between 0.1% and 10.0% together with 2-(2-ethoxyethoxy)ethanol at a concentration between 20.0% and 50.0% by total weight of the composition.

The invention also describes a process for the preparation of the composition and said composition for use in the treatment of pathologies wherein a controlled release of the active principle is useful. The composition comprises one or more pharmaceutical active ingredients and it may be administered by parenteral, subcutaneous, vaginal, buccal, oral, topical or rectal route. The composition can be administered by the use of appropriate medical devices.

### State of the art

WO 2009/028764 describes a gel for the release of proteins obtained from a complex of macromolecules with a thermo-reversible polysaccharide in the presence of a salt with salting-out properties. The resulting formulation is liquid at room temperature turning into a gel when injected, from the gel the protein is released.

WO 1999/059549 describes injectable hydrogels based on alginate esters containing a therapeutic protein and a metal ion.

US 2002/0019336 describes a composition containing a mucopolysaccharide, such as chondroitin sulfate or hyaluronate, the composition is useful for the release of proteins and the formation of the gel occurs as the pH value changes.

Miyazaki S. et al. in J Contr Rel 56, 75, 1998 describes the release of indomethacin from thermo-reversible gels obtained by enzyme-partially degraded xyloglucan at concentrations between 1% and 2% by weight in water or phosphate buffer at pH 7.2. Kant A. et al in Pharmacology online 2: 28, 2011 describes the *in situ* formation of gels in which the formation of the gel depends on changes in temperature, pH, presence of ions and ultraviolet rays, enabling the release of an active principle in a controlled way. The gel can be formed with synthetic or natural polymers and can be administered by oral, ocular, rectal, vaginal or injectable route. This document reports that when xyloglucan in aqueous solutions is partially degraded with β-galactosidase, the resulting product has the property to be a thermo-reversible gel, wherein the gel formation occurs by cooling down from high temperatures. This phenomenon does not occur with the native xyloglucan.

In situ gels can comprise mucoadhesive polymers for the release of active ingredients into the mucous membranes as described in EP 3173067.

EP 1898876 describes mucoadhesive compositions containing xyloglucan in aqueous solution at a concentration between 0.05% and 5.0% by weight, together with glycerol at concentrations between 10.0% and 70.0% by weight, wherein the mucoadhesive compositions described are in solution, ovules, gel, vaginal lavage and spray. This patent does not describe the formation of *in situ* gel.

JP 6490134 describes gel compositions containing an aqueous solution of xyloglucan and at least one saccharide and/or an alcohol. The described composition comprises xyloglucan at a concentration between 0.1% and 15.0% by weight, an alcohol selected in the group consisting of a di-, tri-, tetra-, penta and hexahydric alcohol in an amount between 10,0% and 50.0% by weight and a water-soluble polymer. The described compositions refer to gel compositions for cosmetic and food use, wherein the aim is to maintain the gel obtained during the storage thereof.

Todaro S. et al. Chem. in Eng. Trans. 2016; 49:289-294, describes the rheological properties of xyloglucan depolymerized with high energy irradiation. The fractions of depolymerized xyloglucan were characterized by FT-IR and by the formation of gel in presence of different concentration of ethanol.

Pasta S. et al., in "I materiali biocompatibili per la medicina/Biomaterials for Medicine" 2014 (2014-01-01), Universitas Studiorum, XP055676486, pages 369-370, describes gel formation when native or depolymerized xyloglucan are mixed with Transcutol^{®} in a relative ratio 4:1 and he states that this gel could be useful in tissue engineering for human application.

In the pharmaceutical field there is an increased need to find new pharmaceutical compositions able of releasing active ingredients in a controlled way over time, such as to maintain the plasma concentration in an effective level resulting in a reduced number of administrations over time.

In view of the above, the object of the present invention is a controlled release pharmaceutical composition in gel form, containing one or more active ingredients. The gel is formed directly at the contact between a natural polymer, xyloglucan, and an alcohol. The pharmaceutical gel composition comprising an active pharmaceutical ingredient can be obtained by mixing the components before use, in situ or for the preparation of a pharmaceutical product to be stored at room temperature or refrigerated.

### Summary of the invention

The present invention relates to a controlled release composition in form of gel comprising a pharmaceutical active ingredient.

This composition comprises xyloglucan, a primary alcohol and one or more active ingredients, according to claim 1.

The invention discloses a controlled release composition in form of gel, comprising one or more pharmaceutical active ingredients, xyloglucan and a primary alcohol, wherein the xyloglucan is at a concentration between 0.1% and 10.0% by total weight of the composition. The primary alcohol is 2-(2-ethoxyethoxy) ethanol (Transcutol ^{®}), at a concentration between 20.0% (w/w) and 50.0% (w/w) by total weight of the composition.

The composition of the invention can comprise one or more pharmaceutical active ingredients selected from the group comprising anti-inflammatory, anti-fungal, antibiotic, mimetic antibiotic, growth factors, disinfectants, anticancer agents, proteins, peptides, humectants, natural ingredients or their mixtures.
The composition is useful for releasing in a controlled way active ingredient with a topical and/or systemic effect.

The invention also describes a process according to claim 10 for obtaining a composition according to claim 1, comprising the preparation of a purified xyloglucan in an aqueous solution at a concentration from 0.1% to 10.0% by weight, with a 2-(2-ethoxyethoxy)ethanol solution at a concentration of 20.0% to 50.0% by weight wherein the active principle can be included in both solutions depending on the solubility.

### Detailed description of the invention

The present invention describes a controlled release composition in form of gel, wherein the gel comprises pharmaceutical active ingredients which are released in a controlled and effective way.

The composition of the present invention is obtained by mixing an aqueous solution of xyloglucan (XG) with an alcohol, **i.e.** with 2-(2-ethoxyethoxy)ethanol. The pharmaceutical gel composition including a pharmaceutical active ingredient, can be obtained by mixing the two components just before use, *in situ* or for the preparation of a pharmaceutical product to be stored at room temperature or refrigerated.

The active pharmaceutical ingredients comprised in the composition, can be included in the aqueous solution of xyloglucan or in the alcoholic solution according to their solubility, without any limitation. The active ingredients can also be stored as such or diluted in aqueous solutions with or without xyloglucan and included in the gel at the time of administration.

The gel composition is formed by mixing xyloglucan and a primary alcohol selected from 2-(2-ethoxyethoxy) ethanol.

Xyloglucan is a polysaccharide derived from tamarind seeds and is composed of a (1-4)-β-D-glucan chain which has (1-6)-α-D-xylose bonds partially replaced by (1-2)-β-D-galactoxylose. Xyloglucan forms thermo-reversible gels in water when partially degraded by β-galactosidase, and the resulting product, in diluted aqueous solutions, has the property of gelling in reversible thermal conditions. Gel formation is possible when galactose removal exceeds 35%. Furthermore, the transition temperature is inversely proportional to the polymer concentration and the galactose removal. Such behavior does not occur with native xyloglucan. Xyloglucan can be obtained by extraction from plants such as the pea plant, soybean, rice, bamboo or tamarind seeds. Xyloglucan derived from tamarind seeds has a molecular weight of approximately 5,000 to 1,000,000 Da. The polymer is preferably obtained by extraction with alkaline solutions and then further purified by extraction in boiling water, centrifugation and sterilizing filtration.

The xyloglucan included in the composition of the present invention is a native xyloglucan, not enzyme-degraded, purified according to the purification process described in EP 1898876, and it is included in a concentration between 0.1% and 10.0% by total weight of the composition.

The gel composition, obtained by mixing an aqueous solution of xyloglucan at a concentration between 0.1% and 10.0% by weight and a primary alcohol selected from 2-(2-ethoxyethoxy) ethanol at concentration between 20.0% and 50.0% by total weight of the composition, releases the active ingredients in a controlled way.

According to the invention, the gel composition is obtained by mixing an aqueous solution of xyloglucan at a concentration between 0.1% and 10.0% by weight and a primary alcohol selected from 2-(2-ethoxyethoxy) ethanol at concentration between 20.0 and 50.0% by total weight of the composition.

The composition can comprise xyloglucan at a concentration between 0.1% and 10.0% by total weight of the composition and 2-(2-ethoxyethoxy) ethanol at a concentration between 20.0% and 30.0% by total weight of the composition.

The composition can comprise xyloglucan at a concentration between 1.0% and 5.0% and 2-(2-ethoxyethoxy) ethanol at a concentration between 20.0% and 50.0% by total weight of the composition.

The composition can comprise xyloglucan at a concentration between 1.0% and 5.0% and 2-(2-ethoxyethoxy) ethanol at a concentration between 20.0% and 30.0% by total weight of the composition.

By varying the amount of xyloglucan and 2-(2-ethoxyethoxy) ethanol in the composition, it is possible to obtain gels in different amount, characterized by different consistency, and able to modulate the release of the active ingredients over time. The composition of the present invention enables to have a flexible matrix useful to provide controlled releases according to the active principle, the pathology to be treated and the delivery site.

It has been found that the addition of a primary alcohol, in particular 2-(2-ethoxyethoxy) ethanol, at a concentration between 20.0% and 50.0% by weight, to the aqueous solution of xyloglucan, at a concentration by weight between 0.1% and 10.0%, forms a gel in less than 20 seconds, preferably from 1 to 10 seconds.

The composition comprises xyloglucan at a concentration between 0.1% and 10.0% by weight, preferably between 0.5% to 8.0% by weight, more preferably between 1.0% and 5.0% by total weight of the composition. The composition comprising xyloglucan at concentrations between 0.1% and 10.0% by weight with 2-(2-ethoxyethoxy) ethanol at concentrations between 20.0% and 50.0% by weight, is characterized by a rheological behavior different in comparison to those obtained by a composition comprising xyloglucan (XG) and secondary alcohols at the same concentrations.

The gel composition comprising xyloglucan at concentrations between 0.1% and 10.0% by weight and 2-(2-ethoxyethoxy) ethanol at concentrations between 20.0% and 50.0% by weight, forms a gel in a percentage by weight greater than 50,0% with respect to the weight of the composition. Furthermore, the amount of gel formed by the composition comprising xyloglucan at concentration between 0.1 and 10.0% and 2-(2-ethoxyethoxy) ethanol between 20.0% and 50.0% is greater than that obtained at the same concentrations from a composition comprising xyloglucan and a secondary alcohol such as for example propylene glycol.

Amounts of xyloglucan less than 0.1% form an amount of gel, is in an amount less than 10.0% with respect to the weight of the solution.

The composition comprising xyloglucan in concentrations between 2.0% and 5.0% by weight and 2-(2-ethoxyethoxy) ethanol in concentrations between 20.0% and 50.0% (w/w) forms a gel separating from the solution, which is in an amount greater than 60.0% of the weight of the solution.

According to the invention the composition can comprise 4% (w/w) xyloglucan and 50% (w/w) 2-(2-ethoxyethoxy) ethanol.

According to the invention the composition can comprise xyloglucan at a concentration between 2.0% (w/w) and 5.0% (w/w) and 2-(2-ethoxyethoxy) ethanol at a concentration between 20.0% (w/w) and 30.0% (w/w) by total weight of the composition.

Compositions comprising xyloglucan at concentrations between 1.0% and 5.0% by weight and a secondary alcohol such as propylene glycol (not according to the invention) at concentrations between 20.0% and 50.0% (w/w) form a gel separating from the solution, in an amount less than 50.0% of the weight of the solution and the gel formation requires a longer time than that with 2-(2-ethoxyethoxy) ethanol.

The gel composition obtained by mixing 2-(2-ethoxyethoxy) ethanol at a concentration of between 20.0% and 50.0% (w/w) with an aqueous solution of xyloglucan, at a concentration between 0.1% and 10.0 % (w/w) is obtained by mixing thereof in less than 20 seconds, preferably less than 10 seconds.

The rheological parameters are useful for defining the characteristics of a liquid or a solid. Namely, the moduli G' and G", respectively known as the Storage Modulus and Loss Modulus, are frequency-dependent material functions. G' is representative of solid behavior, G" of liquid behavior. G' is aligned with deformation, in accordance with a typically elastic behavior, G" is in accordance with a typically liquid behavior.

The composition of the invention in form of gel, is characterized by a Storage Modulus (G'), greater than a Loss Modulus (G").

The composition comprising xyloglucan at a concentration between 0.1% and 10.0% by weight and 2-(2-ethoxyethoxy) ethanol between 20.0% and 50.0% by weight, is characterized by values of G' decreasing as frequency decreases. This feature is maintained also after 24 hours.

The composition of the present invention is characterized at time T0 by G' values between 2000 Pa and 500 Pa with a frequency between 100 rad/sec and 0.1 rad/sec applied.

The composition of the present invention is characterized by G' values after 24 hours (T24) between 3000 Pa and 1000 Pa, with a frequency between 100 rad/sec and 0.1 rad/sec applied.

The composition comprising a concentration of xyloglucan between 0.1% and 10.0% by weight and 2-(2-ethoxyethoxy) ethanol between 20.0% and 50.0% (w/w) is characterized at Time zero (T0) by values of G" between 500 Pa and 100 Pa, when a stress with a frequency between 100 rad/sec and 0.1 rad/sec applied.

The composition comprising a concentration of xyloglucan between 0.1% and 10.0% (w/w) and 2-(2-ethoxyethoxy) ethanol between 20.0% and 50.0% (w/w) is characterized at T24 by values of G" between 300 Pa and 100 Pa, a frequency between 100 rad/sec and 0.1 rad/sec applied.

The composition comprising xyloglucan at the concentrations above described and a secondary alcohol (not according to the invention), such as for example propylene glycol, is characterized by values of G' lower than G" and both lower than those obtained in a composition comprising xyloglucan and 2-(2-ethoxyethoxy) ethanol at T0. The gel containing propylene glycol is formed in a longer time than that containing 2-(2-ethoxyethoxy) ethanol and the formed gel is characterized by weak form. This is confirmed by the values of G' which are lower than those obtained with 2-(2-ethoxyethoxy) ethanol both at T0 and after 24 hours.

The composition comprising xyloglucan at a concentration between 0.1% and 10.0% by weight and 2-(2-ethoxyethoxy) ethanol at a concentration between 20.0% and 50.0% by weight is characterized at T0 by viscosity values greater than those obtained with a composition comprising xyloglucan and propylene glycol at the same concentrations. The composition comprising xyloglucan at a concentration between 0.1% and 10.0% by weight and 2-(2-ethoxyethoxy) ethanol at a concentration between 20.0% and 50.0% by weight, at time T0, is characterized by viscosity values ranging from 1x10¹⁰ to 2x10³ mPa when a shear stress with a shear rate from 0 to 95 sec⁻¹ is applied.

The composition comprising xyloglucan at a concentration from 0.1% to 10.0% by weight and 2-(2-ethoxyethoxy) ethanol at a concentration between 20.0% and 50.0% by weight after 24 hours is characterized by viscosity values between 3x10⁷ and 1x10³ mPa·s when a shear stress with a shear rate of 0 to 95 sec⁻¹ is applied.

A comparative composition comprising xyloglucan and a secondary alcohol, such as propylene glycol, at the same concentrations, at T0 shows viscosity values lower than those obtained in the compositions containing 2-(2-ethoxyethoxy) ethanol. The viscosity values of this composition even after 24 hours are lower than those of the composition containing 2-(2-ethoxyethoxy) ethanol, by at least one order of magnitude. The composition comprising xyloglucan at a concentration between 0.1% and 10.0% by weight and 2-(2-ethoxyethoxy) ethanol between 20.0% and 50.0% by weight, can include other alcohols or esters which can slow down the release of active principles. Such additives can be, for example, natural or synthetic polymers, soluble in water or water-insoluble waxes. These compounds can be in a weight ratio between 1:50 and 10:1 with respect to xyloglucan in the aqueous solution.

The gels are formed when the xyloglucan aqueous solution is mixed with 2-(2-ethoxyethoxy) ethanol. The two solutions can therefore be stored in suitable containers to be used as needed and prepared by mixing xyloglucan and 2-(2-ethoxyethoxy) ethanol to form the gel at the site of action. The two components can be delivered in the desired site by forming in this site the gel able of releasing the active ingredient by the use of a system, or a medical device having two channels or two chambers containing the two solutions. Depending on its solubility in water, the active substance can be included in the aqueous solution of xyloglucan or in the alcoholic solution of 2-(2-ethoxyethoxy) ethanol.

A process for the preparation of the composition according to the invention comprises the steps of:
- preparing an aqueous solution of purified xyloglucan at a concentration from 0.1% to 10.0% by weight, adding the obtained xyloglucan solution to a solution of 2-(2-ethoxyethoxy)ethanol at a concentration from 20.0% to 50.0% by weight, wherein the active ingredient may be comprised in both solutions depending on its solubility.

The solutions are mixed at the time of use, before the use or during the phase of gel preparation to be stored.

The gel composition containing xyloglucan at a concentration between 0.1% and 10.0% by weight and 2-(2-ethoxyethoxy) ethanol between 20.0% and 50.0% by weight and pharmaceutical ingredients, wherein these ingredients are immediately included in the gel favors their stability, avoiding possible problems of degradation. Gel compositions comprising xyloglucan between 0.1% and 10.0% by weight and 2-(2-ethoxyethoxy) ethanol between 20.0% and 50.0% by weight can comprise one or more pharmaceutical active ingredients selected from the group comprising anti-inflammatory, anti-fungal, antibiotics, mimetic antibiotics, growth factors, disinfectants, anticancer agents, proteins, peptides, humectants and natural ingredients or their mixture.

The gel composition is useful for releasing peptides, proteins and any other active ingredient that can degrade when stored if formulated with other excipients.

The administrations can take place by enteral (oral, sublingual, rectal), parenteral (subcutaneous, intradermal), transcutaneous route. The compositions can also be administered vaginally, nasally or through the oropharyngeal mucosa.

For example, this composition can be useful for administering drugs that easily reach the affected site for example by inflammation or infection, injury, dehydration. The compositions may also include, for example, recombinant proteins such as human recombinant interferon (rIFN), rIFN alpha, rIFN beta and rIFN gamma, G-CSF, wherein the release takes place in a controlled manner with a total recovery of the administered amount. The ability of the composition according to the invention to release an active principle in a controlled way has been demonstrated by an exemplary experiment using rIFN α2b as active principle, as below described. The amount of active IFN released by the system was measured after 1, 3, 5, 24 and 48 hours.

Measurements have shown that after 5 hours the total amount of IFN released was about 26 %, after 24 hours the total amount of IFN released was about 60 %, and after 48 hour the IFN is fully released. Such release occurring in 24 and 48 hours enables to always have an effective amount of plasma concentration of the active principle with fewer administrations.

The invention also provides kits for delivering the methods described herein. The kits include a dose of therapeutic agents in suitable packaging.

### Examples

### Reference Example 1: Determination of xyloglucan gel formation with 2-(2-ethoxyethoxy) ethanol

Aqueous solutions of xyloglucan at concentrations of 0.4%, 1.0%, 3.2% and 4.0% by weight were prepared.

The xyloglucan solutions were prepared by mixing the necessary amount of xyloglucan in water and leaving under stirring until completely dissolved.

The xyloglucan solutions were mixed with 2-(2-ethoxyethoxy) ethanol at the concentrations shown in the table, in graduated tubes. In less than 10 seconds after the mixing the two liquids, the formation of the gel was observed. Further to a visual evaluation, the gel amount was determined after centrifugation of the solution and the percentage of gel in the solution was assessed.

The following table shows the relative quantities of the components: xyloglucan, water and 2-(2-ethoxyethoxy) ethanol present in the solution and the amount of gel formed after mixing. The percentage of formed gel is obtained with respect to the total amount of the solution.

**Table 1**

| % Xyloglucan (w/w) | % Water (w/w) | % 2-(2-ethoxyethoxy) ethanol (w/w) | % Gel (w/w) |
|---|---|---|---|
| 0.38 | 94.62 | 5 | 1.05 |
| 0.3 | 74.7 | 25 | 28.7 |
| 0.2 | 49.8 | 50 | 33.3 |
| 0.1 | 24,9 | 75 | 20.15 |
| 0.02 | 4.98 | 95 | 6.8 |
| 0.95 | 94.05 | 5 | 15.2 |
| 0.75 | 74.25 | 25 | 54.4 |
| 0.5 | 49.5 | 50 | 54.45 |
| 0.25 | 24.75 | 75 | 30.55 |
| 0.05 | 4.95 | 95 | 13.25 |
| 3.04 | 91.96 | 5 | 16.6 |
| 2.4 | 72.6 | 25 | 88.15 |
| 1.6 | 48.4 | 50 | 50.55 |
| 0.8 | 24.2 | 75 | 23.3 |
| 0.16 | 4.84 | 95 | 8.35 |
| 3.8 | 91.2 | 5 | 40 |
| 3 | 72 | 25 | 64.9 |
| 2 | 48 | 50 | 44.1 |
| 1 | 24 | 75 | 21.55 |
| 0.2 | 4.8 | 95 | 5.2 |

### Example 2: Determination of xyloglucan gel formation with glycol propylene (Comparative example)

Aqueous solutions of xyloglucan were prepared at concentrations of 0.4%, 1.0%, 3.2% and 4.0% by weight. The solutions were mixed with propylene glycol at the concentrations shown in the table. The xyloglucan solutions were made by mixing the necessary amount of xyloglucan in water and mixing until complete dissolution.

The xyloglucan solutions were mixed with propylene glycol at the concentrations shown in the table, in graduated tubes. Further to a visual evaluation, the gel amount was determined after centrifugation of the solution and the percentage of gel in the solution was assessed.

The following table reports the relative amounts of the components: xyloglucan, water and propylene glycol in the solution and the quantity of gel formed after mixing. The percentage of the formed gel is obtained with respect to the total amount of the solution.

**Table 2**

| % Xyloglucan (w/w) | % Water (w/w) | % Propylene glycol (w/w) | % Gel (w/w) |
|---|---|---|---|
| 0.38 | 94.62 | 5 | 2 |
| 0.3 | 74.7 | 25 | 19.1 |
| 0.2 | 49.8 | 50 | 37.65 |
| 0.1 | 24.9 | 75 | 35.35 |
| 0.02 | 4.98 | 95 | 11.95 |
| 0.95 | 94.05 | 5 | 7.5 |
| 0.75 | 74.25 | 25 | 33.3 |
| 0.5 | 49.5 | 50 | 44.55 |
| 0.25 | 24.75 | 75 | 30.75 |
| 0.05 | 4.95 | 95 | 12.5 |
| 3.04 | 91.96 | 5 | 33.7 |
| 2.4 | 72.6 | 25 | 56.25 |
| 1.6 | 48.4 | 50 | 70.5 |
| 0.8 | 24.2 | 75 | 31.5 |
| 0.16 | 4.84 | 95 | 6.1 |
| 3.8 | 91.2 | 5 | 27.6 |
| 3 | 72 | 25 | 60.8 |
| 2 | 48 | 50 | 59.85 |
| 1 | 24 | 75 | 33.1 |
| 0.2 | 4.8 | 95 | 7.55 |

### Reference Example 3: Determination of the modules G' and G" of the composition xyloglucan-2-(2-ethoxyethoxy) ethanol

A volume of 40 ml of the aqueous solution of xyloglucan at concentration of 3.2% (w/w) was mixed with 10 ml 2-(2-ethoxyethoxy) ethanol. The solutions were loaded onto two syringes and directly extruded onto the rheometer plate and analyzed. The measurements of G' and G" were obtained by an Antoon Paar MCR101 rheometer conditioned at a temperature of 37 °C, using a 50 mm flat cone. The measurements of G' and G" were performed at T0 and T24.

Table 3 reports the values of the modules G' and G" as the applied stress increases.

**Table 3**

| Frequency | Storage modulus G' (T0) | Loss Modulus G" (T0) | Storage modulus G' (T24) | Loss Modulus G" (T24) |
|---|---|---|---|---|
| [rad/s] | [Pa] | [Pa] | [Pa] | [Pa] |
| 100 | 1150 | 365.04 | 1893.4 | 251.88 |
| 63.1 | 1075.5 | 320.81 | 1828.5 | 233.08 |
| 39.8 | 1007.4 | 280.35 | 1761.2 | 217.48 |
| 25.1 | 951.99 | 239.27 | 1695.8 | 206.21 |
| 15.8 | 896.29 | 204.54 | 1636 | 197.12 |
| 10 | 861.62 | 175.53 | 1577.6 | 191.35 |
| 6.31 | 821.93 | 154.2 | 1521.3 | 185.55 |
| 3.98 | 802.85 | 129.89 | 1469.4 | 184.7 |
| 2.51 | 781.69 | 120.13 | 1414.8 | 181.28 |
| 1.58 | 766.91 | 113.52 | 1364.4 | 177.65 |
| 1 | 764.91 | 104.86 | 1308.3 | 180.72 |
| 0.631 | 766.91 | 100.94 | 1258 | 180.29 |
| 0.398 | 768.6 | 107.59 | 1220.1 | 183.13 |
| 0.251 | 778.46 | 106.41 | 1168 | 182.77 |
| 0.158 | 815.9 | 109.97 | 1138 | 170.4 |
| 0.1 | 1104.5 | 120.44 | 1112.2 | 166.16 |

### Example 4: Determination of Storage Modulus (G') and Loss Modulus (G") of the composition containing xyloglucan and propylene glycol (Comparative example)

The Example is carried out as in Example 3 and the values of Storage Modulus (G') and Loss Modulus (G") of the composition obtained by mixing 40 ml of the aqueous solution 3.2% (w/w) xyloglucan with 10 ml of 2-(2-ethoxyethoxy) ethanol were determined.

Table 4 shows the values of the modules G' and G" measured at time T0 and after 24 hours (T24).

**Table 4**

| Frequency | Storage Modulus G' (T0) | Loss Modulus G" (T0) | Storage Modulus G' (T24) | Loss Modulus G" (T24) |
|---|---|---|---|---|
| [rad/s] | [Pa] | [Pa] | [Pa] | [Pa] |
| 100 | 142.03 | 175.29 | 837.43 | 170.62 |
| 63.1 | 96.606 | 141.05 | 799.77 | 154.67 |
| 39.8 | 62.894 | 109.66 | 760.56 | 139.13 |
| 25.1 | 39.255 | 82.484 | 723.26 | 125.54 |
| 15.8 | 23.506 | 60.17 | 687.88 | 114.27 |
| 10 | 13.355 | 42.736 | 658.45 | 102.08 |
| 6.31 | 7.1791 | 29.591 | 631.71 | 93.689 |
| 3.98 | 3.8704 | 19.83 | 602.17 | 89.072 |
| 2.51 | 1.9388 | 13.13 | 581.87 | 81.43 |
| 1.58 | 0.95303 | 8.5891 | 558.19 | 75.859 |
| 1 | 0.4636 | 5.5399 | 539.03 | 71.59 |
| 0.631 | 0.23673 | 3.5711 | 515.81 | 75.731 |
| 0.398 | 0.12583 | 2.2806 | 502.23 | 66.3 |
| 0.251 | 0.070546 | 1.4495 | 484.94 | 62.991 |
| 0.158 | 0.029126 | 0.93052 | 474.18 | 63.64 |
| 0.1 | 0.029699 | 0.59432 | 463.08 | 68.4 |

### Reference Example 5: Determination of the viscosity of compositions comprising xyloglucan and 2-(2-ethoxyethoxy) ethanol

The viscosity values were obtained by an Antoon Paar MCR101 rheometer, with a 50 mm flat cone geometry and the measurements were performed at 37 °C. Table 5 reports the viscosity values of the composition obtained by mixing 40 ml of the 3.2% (w/w) xyloglucan aqueous solution with 10 ml 2-(2-ethoxyethoxy) ethanol at T0 and after 24 hours.

**Table 5**

| Shear -rate (1/s) | Viscosity (mPa·s) | |
|---|---|---|
| | T0 | T24 |
| 0.00958 | 1.50 x10⁷ | 2.20 x10⁷ |
| 0.0172 | 6.44 x10⁶ | 1.30 x10⁷ |
| 0.0296 | 3.58 x10⁶ | 7.42 x10⁶ |
| 0.0509 | 2.14 x10⁶ | 4.21 x10⁶ |
| 0.0875 | 1.28 x10⁶ | 2.41 x10⁶ |
| 0.15 | 7.83 x10⁵ | 1.41 x10⁶ |
| 0.258 | 4.82 x10⁵ | 8.45 x10⁵ |
| 0.444 | 2.98 x10⁵ | 5.25 x10⁵ |
| 0.764 | 1.77 x10⁵ | 3.31 x10⁵ |
| 1.31 | 1.15 x10⁵ | 2.12 x10⁵ |
| 2.26 | 69493 | 1.34 x10⁵ |
| 3.88 | 44152 | 83993 |
| 6.69 | 27064 | 48581 |
| 11.5 | 16767 | 26180 |
| 19.8 | 10496 | 12586 |
| 34 | 6678.7 | 5623.9 |
| 56.2 | 4541.5 | 2649.2 |
| 94.3 | 2995.3 | 1350 |

### Example 6: Determination of the viscosity of compositions comprising xyloglucan and propylene glycol (Comparative example)

The viscosity values were obtained by an Antoon Paar MCR101 rheometer, with a 50 mm flat cone geometry and the measurements were performed at 37 °C.

Table 6 reports the viscosity values of the composition obtained by mixing 40 ml of the 3.2% (w/w) aqueous solution of xyloglucan with 10 ml of propylene glycol at time T0 and after 24 hours.

**Table 6**

| Shear rate (1/s) | Viscosity (mPa·s) | |
|---|---|---|
| | T0 | T24 |
| 0.00991 | 1.12x10⁶ | 4.46 x10⁶ |
| 0.0171 | 1.01 x10⁶ | 3.39 x10⁶ |
| 0.0295 | 8.67 x10⁵ | 2.30 x10⁶ |
| 0.0507 | 7.09 x10⁵ | 1.55 x10⁶ |
| 0.0871 | 4.52 x10⁵ | 1.04 x10⁶ |
| 0.152 | 2.79 x10⁵ | 4.94 x10⁵ |
| 0.258 | 1.56 x10⁵ | 3.39 x10⁵ |
| 0.444 | 87819 | 2.30 x10⁵ |
| 0.763 | 55029 | 1.55 x10⁵ |
| 1.31 | 35316 | 1.04 x10⁵ |
| 2.26 | 21461 | 68641 |
| 3.88 | 13608 | 44952 |
| 6.67 | 8996 | 28991 |
| 11.5 | 6152 | 18854 |
| 19.7 | 4050.8 | 12152 |
| 33.9 | 2672.9 | 7873.8 |
| 55.7 | 2037.6 | 5419.7 |
| 93.8 | 1526.8 | 3768.2 |

### Example 7: Determination of the release of recombinant interferon (rIFN α2b) from xyloglucan-2-(2-ethoxyethoxy) ethanol gel

A recombinant IFN α2b solution, containing 2.6x10⁸ IU/mg was prepared in a 4% by weight xyloglucan solution at pH 7; the unitary composition is reported in Table 7.

**Table 7**

| Component | Unitary composition unitaria per 1 g | Composition % (w/w) |
|---|---|---|
| Na₂HPO₄ | 1.3 mg | 0.13 |
| NaH₂PO₄ H₂O | 1.8 mg | 0.18 |
| Glycine | 10 mg | 10 |
| Tween 80 | 0.1 mg | 0.01 |
| Xyloglucan | 40 mg | 4 |
| H₂O | q.s. to 1 g | |
| rrIFNα2b (2.6x10⁸ IU/mg) | 0.096 ml | 0.0096 |
| pH 7 | | |

rIFN α2b in the gel has a concentration of 25,000,000 IU/ml, so that 20,000,000 IU are present in the extruded gel.

In a double chamber syringe, 2 ml of solution containing xyloglucan according to Table 7 were loaded in one channel and 0.5 ml of 2-(2-ethoxyethoxy) ethanol was loaded in the other one. Following the extrusion from the syringe, a gel is immediately formed, in less than 10 seconds.

To evaluate the release of IFN, the gel was placed in 2% (w/w) culture medium (MEM), thermostated at 37 °C and assessed by a biological test for cytopathic effect (CPE).

**Table 8**

| Time (hrs) | IU/ml | Total IU | Released IU/h |
|---|---|---|---|
| 0 | 3,906 | 78,113 | / |
| 1 | 64,471 | 1,293,327 | 1,293,327 |
| 3 | 226,480 | 4,597,970 | 1,532,657 |
| 5 | 291,977 | 6,134,405 | 1,226,881 |
| 24 | 672,360 | 14,034,027 | 584,751 |
| 48 | 1,097,957 | 23,218,341 | 483,715 |
| 72 | 755,755 | 17,472,241 | 242,670 |
| 96 | 899,449 | 21,101,890 | 219,811 |
| 168 | 842,355 | 20,859,456 | 124,163 |

In vitro release kinetics indicate that IFN is fully released between 24 and 48 hours, with 100% recovery.

## Claims

1. A controlled release composition in the form of gel comprising a pharmaceutical active ingredient, xyloglucan at a concentration between 0.1% (w/w) and 10.0% (w/w) and 2-(2-ethoxyethoxy) ethanol (Transcutol^{®}) at a concentration between 20.0 % (w/w) and 50.0 % (w/w) by total weight of the composition.

2. The composition according to claim 1, wherein 2-(2-ethoxyethoxy) ethanol is at a concentration between 20.0% (w/w) and 30.0% (w/w), by total weight of the composition.

3. The composition according to anyone of claims 1 and 2, wherein the xyloglucan is at a concentration between 1.0% (w/w) and 5.0% (w/w) and 2-(2-ethoxyethoxy) ethanol is at a concentration between 20.0% (w/w) and 30.0% (w/w) by total weight of the composition.

4. The composition according to claim 1 comprising 4% (w/w) xyloglucan and 50.0 % (w/w) 2-(2-ethoxyethoxy) ethanol.

5. The composition according to claims 1-4 **characterized by** a Storage Modulus (G') value between 2000 Pa and 500 Pa at T0, and by a value between 3000 Pa and 1000 Pa after 24 hours, when subjected to a frequency between 100 rad/sec and 0.1 rad/sec.

6. The composition according to claims 1-5, **characterized by** a viscosity value between 1x10¹⁰ mPa·s and 2x10³ mPa·s at T0 and by a viscosity value between 3x10⁷ mPa·s and 1x10³ mPa·s at T24, when subjected to a shear rate between 0 sec⁻¹ and 95 sec⁻¹.

7. The composition according to claim 1 wherein the pharmaceutical active ingredient is selected from the group consisting of anti-inflammatory, antifungal, antibiotics, mimetic antibiotics, growth factors, disinfectants, anti-tumorals, proteins, peptides, humectants.

8. The composition according to claim 1 for administration by enteral, parenteral, transcutaneous or transmucosal route.

9. The composition according to claim 8 wherein the administration by enteral route is oral, sublingual and rectal administration, the administration by parenteral route is subcutaneous or intradermic administration, the administration by transcutaneous or transmucosal route is through vaginal, nasal or oropharyngeal mucosa.

10. A process for the preparation of the composition according to claim 1 comprising the steps of:
a) preparation of an aqueous solution of purified xyloglucan at a concentration between 0.1% (w/w) and 10.0% (w/w);
b) addition of the xyloglucan solution of step a) to a solution of 2-(2-ethoxyethoxy)ethanol at a concentration between 20.0% (w/w) and 50.0% (w/w), wherein the active ingredient may be comprised in both solutions depending on its solubility.

11. The process according to claim 10 wherein the solutions are mixed at the time of use, before the use or during the phase of gel preparation to be stored.

12. The composition according to claim 1 for use in the treatment of pathologies wherein a controlled release of the active ingredient is useful.

13. The composition according to claim 1 comprised in a medical device.

## Patentansprüche

1. Eine Zusammensetzung mit kontrollierter Freisetzung in Form eines Gels, die einen pharmazeutischen Wirkstoff, Xyloglucan in einer Konzentration zwischen 0,1% (w/w) und 10,0% (w/w) und 2-(2-Ethoxyethoxy)ethanol (Transcutol^{®}) in einer Konzentration zwischen 20,0% (w/ w) und 50,0% (w/w) des Gesamtgewichts der Zusammensetzung enthält.

2. Die Zusammensetzung gemäß Anspruch 1, wobei 2-(2-Ethoxyethoxy)ethanol in einer Konzentration zwischen 20,0% (w/w) und 30,0% (w/w), bezogen auf das Gesamtgewicht der Zusammensetzung, vorliegt.

3. Die Zusammensetzung gemäß einem der Ansprüche 1 und 2, wobei das Xyloglucan in einer Konzentration zwischen 1,0% (w/w) und 5,0% (w/w) und 2-(2-Ethoxyethoxy)ethanol in einer Konzentration zwischen 20,0% (w/w) und 30,0% (w/w) des Gesamtgewichts der Zusammensetzung vorliegt.

4. Die Zusammensetzung gemäß Anspruch 1, umfassend 4 % (w/w) Xyloglucan und 50,0 % (w/w) 2-(2-Ethoxyethoxy)ethanol.

5. Die Zusammensetzung gemäß den Ansprüchen 1 bis 4 ist **gekennzeichnet durch** einen Speichermodulwert (G') zwischen 2000 Pa und 500 Pa bei T0 und durch einen Wert zwischen 3000 Pa und 1000 Pa nach 24 Stunden, wenn sie einer Frequenz zwischen 100 rad/s und 0,1 rad/s ausgesetzt wird.

6. Die Zusammensetzung gemäß den Ansprüchen 1 bis 5, **gekennzeichnet durch** einen Viskositätswert zwischen 1x10¹⁰ mPa·s und 2x10³ mPa·s bei T0 und einem Viskositätswert zwischen 3x10⁷ mPa·s und 1x10³ mPa-s bei T24, wenn es einer Schergeschwindigkeit zwischen 0 s ⁻¹ und 95s ⁻¹ ausgesetzt ist.

7. Die Zusammensetzung gemäß Anspruch 1, wobei der pharmazeutische Wirkstoff aus der Gruppe ausgewählt ist, die aus entzündungshemmenden Mitteln, Mitteln gegen Pilzinfektionen, Antibiotika, mimetischen Antibiotika, Wachstumsfaktoren, Desinfektionsmitteln, Mitteln gegen Tumore, Proteinen, Peptiden und Feuchthaltemitteln besteht.

8. Zusammensetzung nach Anspruch 1 zur enteralen, parenteralen, transkutanen oder transmukosalen Verabreichung.

9. Zusammensetzung nach Anspruch 8, wobei die enterale Verabreichung eine orale, sublinguale oder rektale Verabreichung ist, die parenterale Verabreichung eine subkutane oder intradermale Verabreichung ist und die transkutane oder transmukosale Verabreichung über die Vaginal-, Nasen- oder Oropharynxschleimhaut erfolgt.

10. Ein Verfahren zur Herstellung der Zusammensetzung gemäß Anspruch 1, das die folgenden Schritte umfasst:
a) Herstellung einer wässrigen Lösung aus gereinigtem Xyloglucan in einer Konzentration zwischen 0,1 % (w/w) und 10,0 % (w/w);
b) Zugabe der Xyloglucan-Lösung aus Schritt a) zu einer Lösung von 2-(2- Ethoxyethoxy)ethanol in einer Konzentration zwischen 20,0% (w/w) und 50,0% (w/w)
wobei der Wirkstoff je nach Löslichkeit in beiden Lösungen enthalten sein kann.

11. Verfahren nach Anspruch 10, wobei die Lösungen zum Zeitpunkt der Verwendung, vor der Verwendung oder während der Phase der Gelherstellung gemischt werden, um sie aufzubewahren.

12. Die Zusammensetzung gemäß Anspruch 1 zur Verwendung bei der Behandlung von Erkrankungen, bei denen eine kontrollierte Freisetzung des Wirkstoffs nützlich ist.

13. Die Zusammensetzung gemäß Anspruch 1 ist in einem medizinischen Gerät enthalten.

## Revendications

1. Composition à libération contrôlée sous forme de gel comprenant un principe actif pharmaceutique, du xyloglucane à une concentration comprise entre 0,1 % (p/p) et 10,0 % (p/p) et du 2-(2-éthoxyéthoxy)éthanol (Transcutol^{®}) à une concentration comprise entre 20,0% (p/ p) et 50,0% (p/p), en poids total de la composition.

2. Composition selon la revendication 1, dans laquelle le 2-(2-éthoxyéthoxy)éthanol est présent à une concentration comprise entre 20,0% (p/p) et 30,0% (p/p), en poids total de la composition.

3. La composition selon l'une quelconque des revendications 1 et 2, dans laquelle le xyloglucane est présent à une concentration comprise entre 1,0 % (p/p) et 5,0 % (p/p) et le 2-(2-éthoxyéthoxy)éthanol est présent à une concentration comprise entre 20,0 % (p/p) et 30,0 % (p/p), en poids total de la composition.

4. La composition selon la revendication 1 comprenant 4 % (p/p) de xyloglucane et 50,0 % (p/p) de 2-(2-éthoxyéthoxy)éthanol.

5. La composition selon les revendications 1 à 4, **caractérisée par** une valeur de module de stockage (G') comprise entre 2000 Pa et 500 Pa à T0, et par une valeur comprise entre 3000 Pa et 1000 Pa après 24 heures, lorsqu'elle est soumise à une fréquence comprise entre 100 rad/sec et 0,1 rad/sec.

6. La composition selon les revendications 1 à 5, **caractérisée par** une valeur de viscosité comprise entre 1x10 ¹⁰ mPa·s et 2x10 ³ mPa·s à T0 et par une valeur de viscosité comprise entre 3x10 ⁷ mPa·s et 1x10 ³ mPa-s à T24, lorsqu'elle est soumise à un taux de cisaillement compris entre 0 sec ⁻¹ et 95 sec ⁻¹.

7. La composition selon la revendication 1, dans laquelle l'ingrédient actif pharmaceutique est choisi dans le groupe constitué par les anti-inflammatoires, les antifongiques, les antibiotiques, les antibiotiques mimétiques, les facteurs de croissance, les désinfectants, les antitumoraux, les protéines, les peptides, les humectants.

8. La composition selon la revendication 1 pour administration par voie entérale, parentérale, transcutanée ou transmucosale.

9. La composition selon la revendication 8, dans laquelle l'administration par voie entérale est une administration orale, sublinguale et rectale, l'administration par voie parentérale est une administration sous-cutanée ou intradermique, l'administration par voie transcutanée ou transmuqueuse est à travers la muqueuse vaginale, nasale ou oropharyngée.

10. Un procédé de préparation de la composition selon la revendication 1, comprenant les étapes suivantes :
a) préparation d'une solution aqueuse de xyloglucane purifié à une concentration comprise entre 0,1% (p/p) et 10,0% (p/p) ;
b) ajout de la solution de xyloglucane de l'étape a) à une solution de 2-(2- éthoxyéthoxy)éthanol à une concentration comprise entre 20,0% (p/p) et 50,0% (p/p)
dans lequel l'ingrédient actif peut être compris dans les deux solutions en fonction de sa solubilité.

11. Le procédé selon la revendication 10, dans lequel les solutions sont mélangées au moment de l'utilisation, avant l'utilisation ou pendant la phase de préparation du gel à stocker.

12. La composition selon la revendication 1 pour une utilisation dans le traitement de pathologies dans lesquelles une libération contrôlée du principe actif est utile.

13. La composition selon la revendication 1 comprise dans un dispositif médical.
